# EUROPEAN PATENT APPLICATION

(11) **EP 2 366 424 A1**
(43) Date of publication of application: **21.09.2011**
(21) Application number: 08877960.8
(22) Date of filing: 05.11.2008
(51) Int. Cl.: A61M 39/02, A61J 1/05, A61M 5/162, A61M 39/00, B01D 39/16

(54) **BREATHABLE STRUCTURE AND METHOD FOR BREATHABLE STRUCTURE FORMATION**

(71) Applicant: JMS Co., Ltd., Hiroshima-shi Hiroshima 730-8652 (JP)
(72) Inventor: KATO, Hidetoshi, Hiroshima-city Hiroshima 730-8652 (JP)
(74) Representative: HOFFMANN EITLE
(86) International application number: PCT/JP2008/070088
(87) International publication number: WO 2010/052765

(57) **Abstract**

Provided is a breathable structure, in which: a luer lock cap (1) is made of synthetic resin having a low melting point, including a through-hole (11) to be covered with a breathable film (2); the breathable film (2) is a two-layered film made of synthetic resin having breathability, in which a second film (22) having a low melting point adheres to a porous first film (21) having water repellency; an auxiliary film body (3) is a two-layered film made of synthetic resin with an outer shape larger than an outer shape of the breathable film (2), in which a fourth film (32) having a melting point higher than a melting point of a third film (31) having a low melting point adheres to the third film (31); the breathable film (2) is connected to the auxiliary film body (3) with the second film (22) being heat-laminated to the third film (31) so that the breathable film (2) does not extend beyond the auxiliary film body (3); and the auxiliary film body (3) is connected to the luer cap lock (1) with an outer edge portion of the third film (31) being heat-laminated to the luer cap lock (1).

## Description

### Technical Field

The present invention relates to a breathable structure, which is adopted in a luer lock cap to be connected to a path of a medical tube body, for example, a liquid medicine tube, and in other housings, and the present invention also relates to a method of forming a breathable structure. In particular, the present invention relates to a highly reliable breathable structure which is easy to produce and a method of forming a breathable structure.

### Background Art

Conventionally, in a polyvinyl chloride (PVC) tube used in medical equipment, an entire circuit needs to be filled with liquid for the purpose of removing air bubbles and foreign matters, imparting hydrophilicity to a tube inner surface, and washing an eluated substance (plasticizer, etc.) out of a tube. At this time, in order to remove air from the circuit, a cap at an end or a three-way stopcock is opened/closed, etc.

Patent Document 1: JP 08-103504 A
Patent Document 2: JP 2002-516160 A
Patent Document 3: JP 06-30995 A

### Disclosure of the Invention

### Problems to be solved by the Invention

However, according to the conventional technology, there are the following problems.
First, at a time of filling with liquid, the cap or the three-way stopcock is opened/closed manually, and hence, there is a fear that liquid leakage may be caused. This may lead to contamination from outside, which necessitates particular caution in medical practice, resulting in the degradation in operability. Further, in the case where liquid is expensive or a strict amount of liquid is supplied, such a sealing/opening system is avoided.

Further, in a number of cases of individual and specific medical treatment, many stopcock portions are operated, and the other operations are also performed. Therefore, the fear of human error, i.e., the failure in operations cannot be eliminated. In this case, there is a problem in that air desired to be removed remains inside.

As a solution to those problems, a cap having a hydrophobic breathable film laminated to its end is known. As the hydrophobic breathable film, a porous sheet obtained by forming polytetrafluoroethylene (PTFE) with high water repellency and high hydrophobicity is used in most cases in the medical field. The fine holes ensure air permeability, and simultaneously the water-repellent force inhibits the liquid invasion into the holes (liquid movement to an opposite side).

Herein, as a cap or the like to be connected to a path of a medical tube body, a polyolefin cap is used considering various characteristics such as connectivity, air tightness, and drug resistance. However, PTFE and polyolefin are different not only in a melting temperature but also in a solubility parameter. Therefore, PTFE and polyolefin cannot adhere to each other merely by heating. Therefore, in the conventional hydrophobic breathable film, a polyolefin non-woven sheet is heat-fused to allow the polyolefin non-woven sheet to invade pores of this PTFE porous sheet to be anchored therein, thereby obtaining a two-layered film, and the non-woven sheet side is laminated to a cap or the like.

However, when the non-woven sheet side is laminated to the cap, a connection force becomes weak in terms of a structure thereof. For example, in the case of pressing heating means against the non-woven sheet, if the pressing force is weak, structural gaps of the non-woven sheet remain, which may cause liquid leakage from a side periphery. In contrast, when the pressing force is strong, the non-woven sheet portion serving as an adhesive scatters, which results in surface connection between PTFE and the cap, which are not originally adhere to each other, thereby decreasing the adhesive strength. Actually, such a breathable cap has low resistance to water pressure during filling with liquid, which causes liquid leakage and peeling. Thus, there is a problem in that high reliability cannot be obtained.

On the contrary, in the case where the cap is to adhere to the PTFE film by placing the PTFE side that is a breathable film on an inner side and the non-woven fabric side made of polyolefin on an outer side, and pressing a die (heating means) from the non-woven fabric side to melt a part of polyolefin of the non-woven fabric, the melted polyolefin sticks to the die, and the breathable film itself cannot adhere to the cap. Even if the breathable film can adhere to the cap temporarily, there is such a problem that the breathable film peels off from the cap when removing the heating means.

Further, it is not easy to develop a new material or change a structure in order to solve the above-mentioned problem, which increases a production cost.

Further, the above-mentioned breathable cap and other housings having breathability are produced integrally by a die and an extruder in most cases. Therefore, the hydrophobic breathable film cannot be heat-laminated easily from outside of the cap or other housings later, which causes constraints on products.

The present invention has been made in view of the above, and has an object to easily provide a highly reliable breathable structure which causes no liquid leakage while ensuring breathability.

### Means for solving the Problems

In order to attain the above-mentioned object, a breathable structure according to claim 1 is a breathable structure adopted in a luer lock cap to be connected to a path of a medical tube body or in other housings, includes: a breathable film; a base portion that is a housing portion in which the breathable film is provided; and an auxiliary film body assisting in connection between the breathable film and the base portion, in which: the base portion is made of synthetic resin having a low melting point, including a through-hole to be covered with the breathable film; the breathable film comprises a two-layered film made of synthetic resin having breathability, in which a second film having a low melting point adheres to a porous first film having water repellency; the auxiliary film body comprises a two-layered film made of synthetic resin, in which a fourth film having a melting point higher than a melting point of a third film having a low melting point adheres to the third film; the breathable film is connected to the auxiliary film body with an inner peripheral portion of the third film being heat-laminated to the second film; and the auxiliary film body is connected to the base portion with an outer peripheral portion of the third film being heat-laminated to the base portion.

Specifically, the invention according to claim 1 is capable of providing a highly reliable breathable structure in which the breathable film is heat-laminated to the auxiliary film body to be integrated therewith and the auxiliary film body is also heat-laminated to the base portion to achieve a sealed state, thus preventing liquid leakage while breathability is ensured. Further, since the water-repellent film (first film) is positioned on a liquid side (housing inner side), the adhesive strength between the first film and the second film is not decreased even if a water pressure is applied, preventing layer separation. In this respect, the reliability of the breathable structure is enhanced.

Further, the fourth film has a melting point higher than that of the third film, the base portion, or the second film. Therefore, for example, even in the case where the auxiliary film body is heat-laminated by pressing heating means, the auxiliary film body does not adhere to the heating means side under control at an appropriate temperature, preventing floating or peeling. Specifically, the breathable structure can be provided easily later from outside of the housing, which can also enhance the productivity.

Note that, in the present application, the low melting points of the second film, third film and base portion refer to those which are at the same level, and the high melting point of the fourth film is higher than these melting points. The melting points of the second film, third film, and base portion are approximate to one another and the compatibility thereof is also high. Therefore, it is preferred that the compositions of the second film, third film, and base portion be identical or approximate to one another. Note that, fluorocarbon resin using water repellency is mainly used for a medical breathable film and has a very high melting point. In terms of the melting point, a relationship: first film>fourth film>(second film, third film, base portion) is obtained. Thus, usually, the melting points of the first and second films are largely different, and the solubility parameters thereof are not approximate to each other. Therefore, as an adhesion method, there is a method of heat-laminating a non-woven sheet so that it is anchored with fine holes of the first film as shown in the conventional technology.

Further, as the third and fourth films, those which are heat-laminated in the usual manner can be used, as long as they are synthetic resins having the melting points different from each other by, for example, about 20°C or more, preferably 30°C or more, and have solubility parameters approximate to each other. In the present application, the breathable film and the auxiliary film body are both two-layered structures. However, they may have a structure of more than two layers as long as the characteristics and functions are not impaired. In this sense, the two-layered structure as used herein includes a structure having two or more layers. Further, in the present application, the "adhesion" between the first and second films or the "adhesion" between the third and fourth films is not limited in its aspect as long as they adhere to each other physically or chemically so that they are not separated, and needless to say, the "adhesion" includes the adhesion by heat lamination.

Further, the inner peripheral portion of the third film refers to an inside (center side) portion of the film, and the outer peripheral portion refers to a film portion on an outer side from the inner peripheral portion.

Further, a breathable structure according to claim 2 is a breathable structure, which is adopted in a luer lock cap to be connected to a path of a medical tube body or in other housings, including: a breathable film; a base portion that is a housing portion in which the breathable film is provided; and an auxiliary film body assisting in connection between the breathable film and the base portion, in which: the base portion is made of synthetic resin including a low melting point, including a through-hole to be covered with the breathable film; the breathable film includes a two-layered film made of synthetic resin having breathability, in which a second film having a low melting point adheres to a porous first film having water repellency; the auxiliary film body includes a two-layered film made of synthetic resin with an outer shape larger than an outer shape of the breathable film, in which a fourth film having a melting point higher than a melting point of a third film having a low melting point adheres to the third film; the breathable film is connected to the auxiliary film body with the second film being heat-laminated to the third film so that the breathable film does not extend beyond the auxiliary film body; and the auxiliary film body is connected to the base portion with an outer edge portion of the third film being heat-laminated to the base portion.

Specifically, the invention according to claim 2 is capable of providing a highly reliable breathable structure in which the breathable film is heat-laminated to the auxiliary film body to be integrated therewith and the auxiliary film body is heat-laminated at a margin outside thereof to the base portion to achieve a sealed state, thus preventing liquid leakage while breathability is ensured. Further, since the water-repellent film (first film) is positioned on a liquid side (housing inner side), the adhesive strength between the first film and the second film is not decreased even if a water pressure is applied, preventing layer separation. In this respect, the reliability of the breathable structure is enhanced.

Further, the fourth film has a melting point higher than that of the third film, the base portion, or the second film. Therefore, for example, even in the case where the auxiliary film body is heat-laminated by pressing heating means, the auxiliary film body does not adhere to the heating means side under control at an appropriate temperature, preventing floating or peeling. Specifically, the breathable structure can be provided easily later from outside of the housing, which can also enhance the productivity. Further, a "lamination margin" is formed merely by setting the auxiliary film body larger than the breathable film. Therefore, the breathable structure can be formed easily without complicating the entire shape and configuration, which can also enhance the productivity.

The outer edge portion of the third film may refer to a portion where the breathable film does not overlap, that is, an area to be called a "lamination margin", and is not necessarily limited to the edge of the outer peripheral circle.

Further, regarding a breathable structure according to claim 3, in the breathable structure according to claim 2, the base portion includes, on a surface thereof: a first recess having substantially the same shape as the outer shape of the auxiliary film body with the through-hole being a center; and a second recess having substantially the same shape as the outer shape of the breathable film at a center of the first recess.

Specifically, the invention according to claim 3 enhances a sealing effect. In addition, by providing a step, the breathable film is accommodated thereon and the tension (swelling) in the thickness direction is not caused. As a result, compared with the case where unnecessary tension is applied at all times to the auxiliary film body due to the swelling of the breathable film portion without providing a step, the reliability of a product is enhanced. Further, for production, positioning becomes easy.

The phrase "substantially the same shape" refers to the shape in which the auxiliary film body or breathable film is accommodated with a slight gap.

Further, regarding a breathable structure according to claim 4, in the breathable structure according to claim 1, 2, or 3, the breathable film is obtained by heat-laminating non-woven fabric of polyolefin to a porous PTFE sheet.

Specifically, in the invention according to claim 4, the breathable structure can be formed using the breathable film that has been used practically and is chemically stable. The water pressure is applied in the thickness direction. Therefore, as the water pressure increases, in particular, the second film made of non-woven fabric of the breathable film becomes dense, which enhances the air tightness between the breathable film and the auxiliary film body to keep the reliability of water tightness and resistance to water pressure.

Further, regarding a breathable structure according to claims 5, in the breathable structure according to any one of claims 1 to 4, the second film, the third film, and the base portion are made of polyolefin.

Specifically, in the invention according to claim 5, conventionally provided members can be used and the invention does not require changes in materials and substantial changes in structure and configuration, which enables a product to be provided at low cost.

It should be noted that, in this application, the fourth film may be made of, for example, a polyester, in particular, polyethylene terephthalate (PET). A polyamide-based resin may be applicable as well. In the case where a polyester is used for the fourth film and a polyolefin is used for the third film, as described above, the polyester and the polyolefin are each selected so as to achieve the following combination. That is, the melting point of the fourth film is higher than that of the third film by about 20°C or more, preferably 30°C or more. Further, a wide range of fluorocarbon resins may be used as the first film. The fluorocarbon resins may be exemplified by a tetrafluoroethylene-perfluoroalkyl vinyl ether copolymer (PFA), a tetrafluoroethylene-hexafluoropropylene copolymer (FEP), a tetrafluoroethylene-ethylene copolymer (ETFE), polyvinylidene difluoride (PVDF), polychlorotrifluoroethylene (PCTFE), and a chlorotrifluoroethylene-ethylene copolymer (ECTFE) as well as PTFE. In terms of workability, strength, and actual use in medical practice, a material to be substantially selected as the polyolefin is, for example, polypropylene (PP) or polyethylene (PE).

Further, regarding a breathable structure according to claim 6, in the breathable structure according to any one of claims 1 to 5, the auxiliary film body is formed by punching out a hole having a shape smaller than the outer shape of the breathable film from the auxiliary film body at a center thereof.

Specifically, the invention according to claim 6 ensures breathability easily while ensuring air tightness and also enhances a sealing effect by setting the auxiliary film body without breathability in a donut shape (annular shape).

Further, a method of forming a breathable structure according to claim 7 is a method of forming a breathable, which is adopted in a luer lock cap to be connected to a path of a medical tube body or other housings, the breathable structure including: a breathable film that is a two-layered film made of synthetic resin having breathability, in which a second film having a low melting point adheres to a porous first film having water repellency; a base portion made of synthetic resin having a low melting point, which is a housing portion including a through-hole to be covered with the breathable film; and an auxiliary film body that is a two-layered film made of synthetic resin, in which a fourth film having a melting point higher than a melting point of a third film having a low melting point adheres to the third film, and which assists in connection between the breathable film and the base portion and has an outer shape larger than an outer shape of the breathable film, the method including: placing the breathable film on the through-hole so that the first film side faces the through-hole, and further placing the auxiliary film body on the breathable film so that the fourth film side is directed upward and the breathable film does not extend beyond the auxiliary film body; and pressing heating means from outside of the housing to heat-laminate the second film to the third film and the third film to the base portion.

Specifically, the invention according to claim 7 is capable of providing a highly reliable breathable structure in which the breathable film is covered with the auxiliary film body by heat lamination so as to be integrated therewith, and the auxiliary film body is heat-laminated at the margin thereof to the base portion to achieve a sealed state, thus preventing liquid leakage while the breathability is ensured. Further, since the water-repellent film (first film) is positioned on the liquid side (housing inner side), the adhesive strength between the first film and the second film is not decreased even if a water pressure is applied, preventing layer separation. Inthis respect, the reliability of the breathable structure is enhanced.

Further, the fourth film has a melting point higher than that of the third film, the base portion, or the second film. Therefore, the auxiliary film body does not adhere to the heating means side under control at an appropriate temperature, preventing floating or peeling. Thus, a breathable structure with high yield and high connection reliability (reliability of resistance to pressure) can be formed. The breathable structure can be formed easily later from outside of the housing, which can also enhance the productivity.

Further, in the method of forming a breathable structure according to claim 7, a method of forming a breathable structure according to claim 8 includes: providing a first recess having substantially the same shape as the outer shape of the auxiliary film body on a surface of the base portion with the through-hole being a center, and providing a second recess having substantially the same shape as the outer shape of the breathable film at a center of the first recess; placing the breathable film on the second recess so that the first film side is directed downward; placing the auxiliary film body on the first recess so that the fourth film side is directed upward; and pressing the heating means from the fourth film side to heat-laminate the second film to the third film and the third film to the base portion.

Specifically, the invention according to claim 8 facilitates the positioning of the breathable film and the auxiliary film body and enhances the productivity. Further, the invention according to claim 8 prevents a shift between the breathable film and the auxiliary film body, which can enhance the yield. Further, the sealing effect is enhanced.

Further, in the method of forming a breathable structure according to claim 7 or 8, a method of forming a breathable structure according to claim 9 includes: firstly heat-laminating the third film to the base portion; and secondly heat-laminating the second film to the third film.

Specifically, in the invention according to claim 9, by performing heat lamination from outside, the expansion, sheet deformation, and floating in lamination are regulated, and thus, heat lamination can be performed reliably on an outer side and an inner side. This can enhance the reliability.

Further, a method of forming a breathable structure according to claim 10 is a method of forming a breathable structure, which is adopted in a luer lock cap to be connected to a path of a medical tube body or in other housings, the breathable structure including: a breathable film that is a two-layered film made of synthetic resin having breathability, in which a second film having a low melting point adheres to a porous first film having water repellency; a base portion made of synthetic resin having a low melting point, which is a housing portion including a through-hole to be covered with the breathable film; and an auxiliary film body that is a two-layered film made of synthetic resin, in which a fourth film having a melting point higher than a melting point of a third film having a low melting point adheres to the third film, and which assists in connection between the breathable film and the base portion and has an outer shape larger than an outer shape of the breathable film, the method including: placing a laminate, in which the second film of the breathable film adheres to the third film of the auxiliary film body so that the breathable film does not extend beyond the auxiliary film body, on the through-hole so that the first film side faces the through-hole; and pressing heating means from outside of the housing to heat-laminate the third film to the base portion.

Specifically, the invention according to claim 10 is capable of providing a highly reliable breathable structure in which the sealing with the base portion can be performed using a margin of an outer edge of the auxiliary film body integrated with the breathable film, thus preventing liquid leakage while the breathability is ensured. Further, since the water-repellent film (first film) is positioned on the liquid side (housing inner side), the adhesive strength between the first film and the second film is not decreased even if a water pressure is applied, preventing layer separation. In this respect, the reliability of the breathable structure is enhanced.

Further, the fourth film has a melting point higher than that of the third film, the base portion, or the second film. Therefore, the auxiliary film body does not adhere to the heating means side under control at an appropriate temperature, preventing floating or peeling. Thus, a breathable structure with high yield and high connection reliability (reliability of resistance to pressure) can be formed. The breathable structure can be formed easily later from outside of the housing, which can also enhance the productivity.

Further, in the method of forming a breathable structure according to claim 10, a method of forming a breathable structure according to claim 11 includes: providing a recess having substantially the same shape as the outer shape of the auxiliary film body on a surface of the base portion with the through-hole being a center; placing the auxiliary film body on the recess so that the fourth film side is directed upward; and pressing the heating means from the fourth film side to heat-laminate the third film to the base portion.

Specifically, the invention according to claim 11 facilitates the positioning of the auxiliary film body and enhances the productivity. Further, the invention according to claim 11 prevents a shift of the auxiliary film body, which can enhance the yield.
Further, the sealing effect is enhanced.

Further, in the method of forming a breathable structure according to claim 11, a method of forming a breathable structure according to claim 12 includes: further providing a second recess having substantially the same shape as the outer shape of the breathable film in the recess so that the through-hole is positioned at a center of the second recess; placing the breathable film on the second recess so that the first film side is directed downward; and pressing the heating means from the fourth film side to heat-laminate the third film to the base portion.

Specifically, the invention according to claim 12 facilitates the positioning of the auxiliary film body and enhances the productivity. Further, the invention according to claim 12 prevents a shift of the auxiliary film body, which can enhance the yield.
Further, the sealing effect is enhanced.

Further, in the method of forming a breathable structure according to any one of claims 7 to 12, regarding a method of forming a breathable structure according to claim 13, the breathable film is obtained by heat-laminating non-woven fabric of polyolefin to a porous PTFE sheet.

Specifically, in the invention according to claim 13, the breathable structure can be formed using the breathable film that has been used practically and is chemically stable. The water pressure is applied in the thickness direction. Therefore, as the water pressure increases, in particular, the second film made of non-woven fabric of the breathable film becomes dense, which enhances the air tightness between the breathable film and the auxiliary film body to keep the reliability of water tightness and resistance to water pressure.

Further, in the method of forming a breathable structure according to any one of claims 7 to 13, regarding a method of forming a breathable structure according to claim 14, the second film, the third film, and the base portion are made of polyolefin.

Specifically, in the invention according to claim 14, conventionally provided members can be used and the invention does not require changes in materials and substantial changes in structure and configuration, which enables a product to be provided at low cost.

### Effects of the Invention

According to the present invention, the highly reliable breathable structure, which prevents liquid leakage while ensuring breathability, can be provided easily. Thus, priming (removal of air) can be performed merely by installing the breathable structure. Further, the breathable structure can be provided easily later from outside of the housing, which can also enhances productivity.

### Best Mode for carrying out the Invention

Hereinafter, an embodiment of the present invention is described in detail with reference to the drawings. Herein, the case where the present invention is applied to a luer lock cap and a breathable structure is formed in an end surface is described.

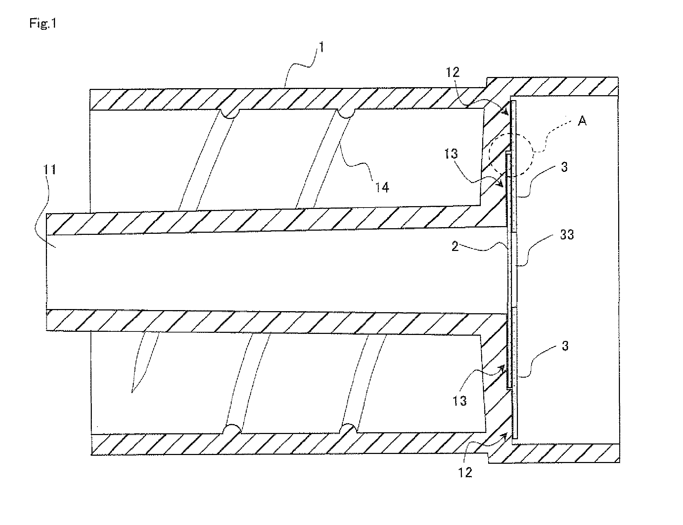

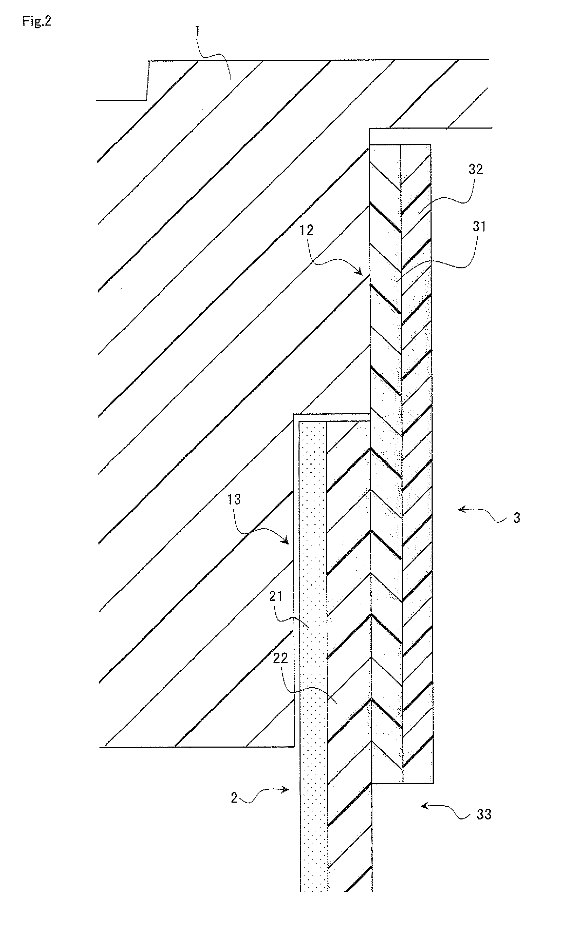

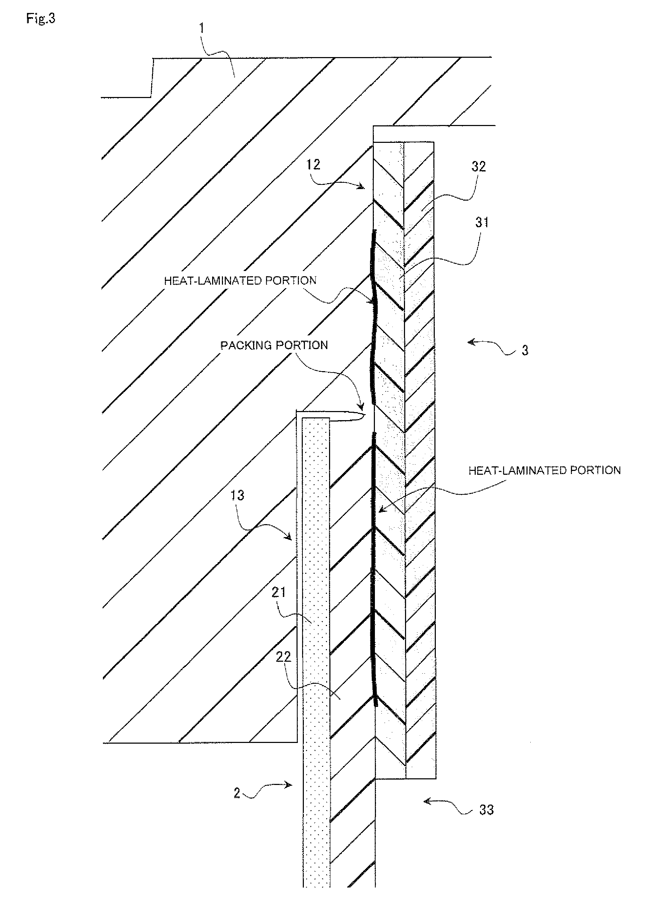

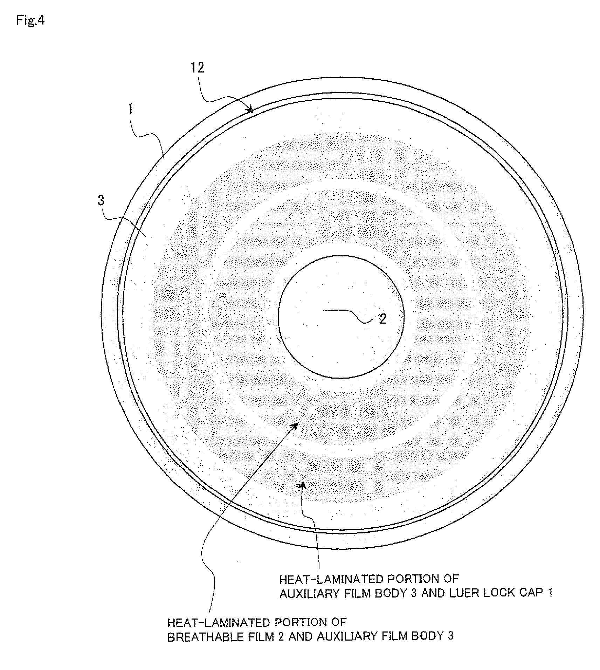

FIG. 1 is a cross-sectional view of a luer lock cap according to this embodiment. FIG. 2 is an enlarged schematic view of a connection portion. FIG. 3 is a conceptual diagram illustrating a state of actual heat lamination. FIG. 4 is a plan view illustrating a state of lamination of an auxiliary film body of the luer lock cap according to this embodiment.

In the breathable structure, a breathable film 2 and an auxiliary film body 3 are placed in this order at a rear end portion of a luer lock cap 1, and the breathable film 2 and the auxiliary film body 3 are heat-laminated and the auxiliary film body 3 and the rear end surface of the luer lock cap 1 are heat-laminated.

The luer lock cap 1 is made of polyolefin, and herein, the luer lock cap 1 made of PP is used. As is apparent from the cross-sectional view, a through-hole 11 is provided at the center of the luer lock cap 1. A circular recess 12 is provided at a cap end portion, and a circular recess 13 is provided on a further inner side. That is, in the rear end surface of the luer lock cap 1, the circular recess 13 as a second step is provided stepwise in the circular recess 12 as a first step so that the centers of the circular recesses 12 and 13 overlap the center of the through-hole 11. The luer lock cap 1 further has a threaded portion 14 formed on an inner surface, and for example, the threaded portion 14 has a shape to be screwed with a three-way stopcock (not shown).

The breathable film 2 is a circular sheet with a diameter slightly smaller than that of the circular recess 13, and is a two-layered film in which a first film 21 made of fluorocarbon resin (here, made of PTFE), which is formed as a fine porous breathable sheet, and a second film 22 made of polyolefin (here, made of PP), which is formed as a non-woven sheet, are heat-laminated to each other. The two-layered film ensures and breathability, water tightness, and adhesion to the auxiliary film body 3. Regarding breathability and water tightness, the breathability, is obtained by the fine porous structure and the water tightness is ensured by preventing liquid invasion with high water repellency of PTFE. Herein, PTFE having a high melting point and PP conversely having a low melting point are not so compatible with each other. Thus, PTFE and PP are unlikely to adhere to each other chemically, and hence, PP is heat-fused to invade the PTFE fine pores, and PTFE and PP are attached to each other physically by anchoring. Further, PP does not have breathability as it is, and hence, the breathability of the second film 22 itself is ensured by forming PP into a non-woven sheet.

More specifically, the breathable film 2 can be produced, for example, by the following method. First, 100 g ot PTFE powder and 26 g of solvent naphtha as a liquid lubricant are mixed. This mixture is pre-formed under a pressure of 50 kg/cm², and extruded by a paste extruder to form a sheet having a desired thickness, e.g., 0.3 mm by rolling. The sheet thus obtained is dried by heating along heating rolls at 200°C to remove the solvent naphtha.

Then, the resultant sheet is stretched by 100% in a uniaxial direction (longitudinal direction) by a roll-type stretching device heated to about 275°C, and further stretched by 200% in the same direction by the roll-type stretching device heated to about 150°C. The stretched sheet is sintered by heating at 400°C for 5 minutes in a state of being stretched, to thereby obtain a porous PTFE sheet. Finally, a nonwoven fabric made of PP and the PTFE sheet are laminated at a predetermined temperature. Finally, the laminate sheet is punched out so as to obtain a circular sheet having a diameter slightly smaller than that of the circular recess 13. Thus, the breathable film 2 having breathability and water tightness is formed.

The auxiliary film body 3 is a donut-shaped two-layered film obtained by punching out a center portion from a circular sheet having a diameter slightly smaller than that of the circular recess 12. Herein, an inner circle to be punched out is smaller than the circle of the breathable film 2, and thus, the connection portion between the auxiliary film body 3 and the breathable film 2 is ensured. Further, breathability is ensured by punching. One surface of the two-layered film is made of polyolefin (herein, made of PP), and the other surface is made of polyester (herein, made of PET). Thereinafter, for convenience, the former is referred to as a third film 31 and the latter is referred to as a fourth film 32. Further, the punched circular portion is referred to as a punched hole 33. PP and PET can be attached to each other by a usual method, for example, a dry lamination method (method in which a diluted adhesive is applied to a film, and two films are attached to each other by take-up).

Although the thickness of a film is not particularly limited, the thickness of the breathable film 2 can be set to, for example, 0.15 mm to 0.35 mm, and the thickness of the auxiliary film body 3 can be set to, for example, 0.10 mm to 0.30 mm. The outer diameter of the luer lock cap 1 is about 10 mm, and the diameter of the through-hole 11 is about 1.5 mm. Therefore, the diameter of the breathable film 2 can be set to 3.0 mm to 6.5 mm, and the outer diameter and the inner diameter of the auxiliary film body 3 can be set to 7. 5 mm to 9. 5 mm and 1.0 mm to 3.5 mm, respectively.

Further, assuming that the outer radius of the auxiliary film body 3, the radius of the breathable film 2, and the inner radius of the auxiliary film body 3 are r₃, r₂, and r₁, respectively, r₃:r₁=6:1 to 2:1 is preferred in terms of breathability, and (r₃-r₂): (r₂-r₁)=2:1 to 1:2 is preferred in terms of an area of heat lamination. It is preferred that the width of heat lamination be 1 mm or more (see FIG. 4). In terms of production, r₃:r₂=5:1 to 5:3, r₂:r₁=7:1 to 7:3 are preferred.

### <Production Example 1>

Next, a method of connecting the circular breathable film 2, the donut-shaped auxiliary film body 3 having an outer diameter larger than and an inner diameter smaller than the diameter of the breathable film 2, and the luer lock cap 1 is described. In Production Example 1, an embodiment is described in which the breathable film 2 and the auxiliary film body 3 are placed separately at the rear end of the luer lock cap 1 and then heat-laminated. In the following example, only portions required in a mass-production line in the industrial production process are described.

First, the luer lock caps 1 are successively arranged with the rear end portions directed upward. For this purpose, the luer lock caps 1 may be held by arms, or there is a method of burying and fixing the luer lock caps 1 in fitting holes of substantially the same shape as the outer shape of the luer lock caps 1. Then, the breathable film 2 is placed on the circular recess 13 with the first film 21 directed downward. For this purpose, there is a method of attracting by a negative pressure the breathable film 2 punched out from the laminate sheet, moving the film, and allowing the film to stand still. This makes it unnecessary to perform determination control of the front/back of the breathable film 2. Further, a great amount of the breathable films 2 are formed at a time by a punching die, and hence, attracting arms may be provided by the number of holes of the die so as to be ready for mass production.

Next, the auxiliary film body 3 is placed on the circular recess 12 with the third film 31 directed downward. Thus, the breathable film 2 is covered with the auxiliary film body 3. Regarding the auxiliary film body 3, using the method similar to the placement of the breathable film 2, a two-layered film with the front/back distinguished immediately after punching can be placed in a correct direction by the attracting arm.

Then, the auxiliary film body 3 is heat-laminated to the circular recess 12. The third film 31 of the auxiliary film body 3 and the circular recess 12 are both made of PP, and thus the third film 31 and the circular recess 12 easily adhere to each other closely. For heating, a cylindrical heater (welding die) slightly smaller than the outer circle of the auxiliary film body 3 is pressed thereagainst for a short period of time, for example, for 3 seconds at 145°C so that the auxiliary film body 3 is inserted in the circular recess 12. Herein, the heater-side fourth film 32 is made of PET. Therefore, the fourth film 32 is not welded to the heater, and when the heater is pulled up, the auxiliary film body 3 does not float or is not taken to the heater side. Thus, strong adhesion can be realized.

Next, the breathable film 2 and the auxiliary film body 3 are heat-laminated. Both the second film 22 of the breathable film 2 and the third film 31 of the auxiliary film body 3 are made of PP, and hence, the second film 22 and the third film 31 easily adhere to each other closely. For heating, a cylindrical heater slightly smaller than the diameter of the breathable film 2 is pressed against the circular recess 13 so as to be inserted therein for a short period of time, for example, for 3 seconds at 145°C. Herein, the second film 22 that is a non-woven sheet is fused from an opposite side of a connection surface with the first film 21, and therefore, the breathable film 2 and the auxiliary film body 3 can be laminated to each other closely without impairing the adhesive strength between the first film 21 and the second film 22.

Finally, the fixing of the luer lock cap 1 is released. By repeating the above-mentioned operations sequentially, luer lock caps with an intended breathable structure formed therein can be obtained.

In an area A illustrated in FIG. 1, the breathable film 2 is to be packed or sealed from a side periphery by fusing PP, which further enhances water tightness (see FIG. 3).

In the above-mentioned example, an embodiment in which the inner peripheral side is heat-laminated after the outer peripheral side is described. The reason for this is as follows. There is a fear that, when the inner peripheral side is heat-laminated first, the film is deformed so as to float upward by heating or pressing to cause looseness and sagging, and the heat lamination on the outer peripheral side is not stabilized, which prevents heat lamination from becoming uniform. Depending upon the control, the outer peripheral side may be heat-laminated after the inner peripheral side. An example in which two heaters having different diameters are used separately is shown. However, the outer and inner peripheral sides may be heat-laminated at a time, using a double cylindrical heater.

### <Production Example 2>

Next, an embodiment is described in which the circular breathable film 2 and the donut-shaped auxiliary film body 3 having the outer diameter larger than and the inner diameter smaller than the diameter of the breathable film 2, which have been heat-laminated in advance, are placed on the luer lock cap 1 and heat-laminated. In the following example, only portions required in a mass-production line in the industrial production process are described.

First, a semifinished product of the auxiliary film body to which the breathable film 2 is heat-laminated is prepared previously. For example, a laminate sheet in which the first film and the second film are heat-laminated is placed on a base with the second film side directed upward, and a circle with a diameter slightly smaller than that of the circular recess 13 is punched out neatly, using a punching frame. Then, the disk sheet (breathable film 2) thus punched out is allowed to remain as it is on the base.

Next, the circular breathable films 2 arranged neatly are covered with a two-layered film sheet of the third and fourth films with the third film side directed downward, and a cylindrical heater with a diameter larger than the diameter of the punched hole 33 is pressed against the breathable film 2 so as to be concentric therewith, to thereby prepare a semifinished product in which a number of the breathable films 2 are heat-laminated to the sheet in the form of spots. The punched holes 33 are formed previously in the two-layered film sheet in which the third and fourth films are connected to each other so as to overlap the respective centers of the circular breathable films 2 arranged neatly on the base.

The cylindrical heater is pressed from the fourth film side. The second film 22 of the breathable film 2 and the third film 31 of the auxiliary film body 3 are both made of PP, and thus the second film 22 and the third film 31 easily adhere to each other closely.

For production, first, the luer lock caps 1 are successively arranged with the rear end portions directed upward. For this purpose, the luer lock caps 1 may be held by arms, or there is a method of burying and fixing the luer lock caps 1 in fitting holes of substantially the same shape as the outer shape of the luer lock caps 1. Then, the auxiliary film body 3, to which the breathable film 2 is heat-laminated, is placed on the circular recess 12 with the first film 21 directed downward. For this purpose, there is a method of attracting by a negative pressure a circular semifinished product having a diameter slightly smaller than that of the circular recess 12 punched out from the semifinished product heat-laminated previously, moving the circular semifinished product, and allowing the circular semi finished product to stand still. This makes it unnecessary to perform determination control of the front/back of the auxiliary film body 3 to which the breathable film 2 is connected. Further, a great amount of the connection films 2 are formed at a time by a punching die, and hence, attracting arms may be provided by the number of holes of the die so as to be ready for mass production.

Then, the auxiliary film body 3 is heat-laminated to the circular recess 12. The third film 31 of the auxiliary film body 3 and the circular recess 12 are both made of PP, and thus the third film 31 and the circular recess 12 easily adhere to each other closely. For heating, a cylindrical heater slightly smaller than the outer circle of the auxiliary film body 3 is pressed thereagainst for a short period of time, for example, for 3 seconds at 145°C so that the auxiliary film body 3 is inserted in the circular recess 12. Herein, the fourth film 32 is made of PET. Therefore, the fourth film 32 is not welded to the heater, and when the heater is pulled up, the auxiliary film body 3 does not float or is not taken to the heater side. Thus, strong adhesion can be realized.

Finally, the fixing of the luer lock cap 1 is released. By repeating the above-mentioned operations sequentially, luer lock caps with an intended breathable structure formed therein can be obtained.

In the above-mentioned two production examples, the placement shift may be detected appropriately, for example, using a sensor.

Further, the second film 22 of the breathable film 2 is a non-woven sheet, and hence is white. Thus, the breathable film 2 becomes white as long as it is not colored. Therefore, if the auxiliary film body 3 is colored, the front/back relationship between the breathable film 2 and the auxiliary film body 3 becomes clear, and hence a product placed in an inverted direction can be detected easily. In a further developed case, the respective layers of the breathable film 2 and the auxiliary film body 3 are colored differently so as to facilitate the detection.

### <Experimental Example>

Next, breathability and resistance to water pressure were evaluated. Using a luer lock cap for an experiment, the present invention was compared with commercially available ones produced by two companies.

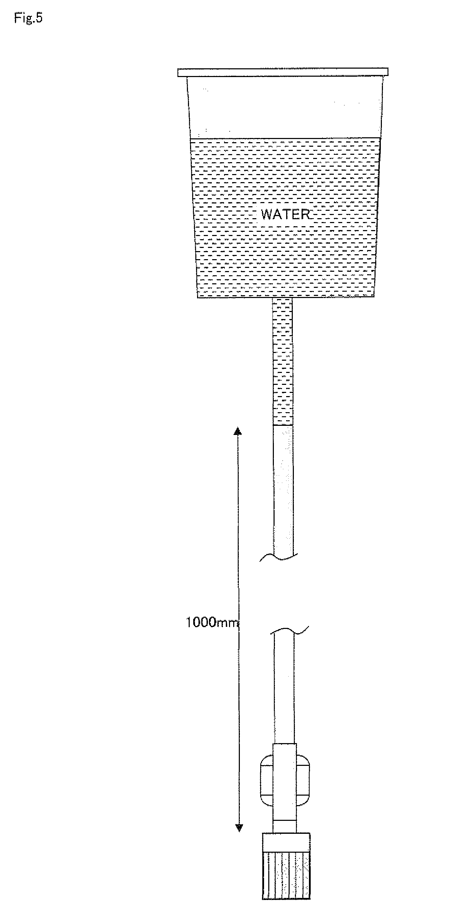

Fig. 5 is an experimental general outline view of an evaluation test of breathability. As illustrated, a bag in which 500 cc of water is loaded is connected from above to a PVC tube with an inner diameter of 3.3 mm, and a luer lock cap is screwed with a lower part of the PVC tube. Herein, a portion of 1,000 mm of the PVC tube above the luer lock cap was adjusted so as to contain air, and the rate at which the air with a height of 1,000 mm was discharged completely is measured. Table 1 shows experimental results. In Table 1, n is the number of experiments.

**[Table 1]**

| | Present Invention | Company A | Company B | Company C |
|---|---|---|---|---|
| Discharge time (second) | 7 to 28 (n=10) | 2.5 to 3.5 (n=5) | 1.8 to 2.2 (n=5) | 23 to 282 (n=4) *There are clogged products |

As shown in the table, sufficient breathability, was confirmed in the present invention. The product of the present invention has a sufficiently practical range although the breathability is lower than those of the companies A and B. On the other hand, some of the products of the company C were clogged, and some products lacking practical breathability were confirmed.

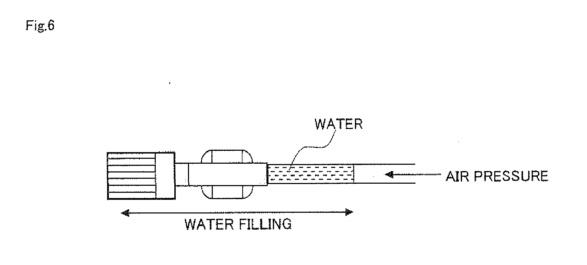

FIG. 6 is an experimental general outline view of an evaluation test of resistance to water pressure. As illustrated, a luer lock cap is connected to a PVC tube, and a portion of the PVC tube on the luer lock cap side is filled with water and pressed with air. As evaluation, a pressure under which water leaks from a rear end surface side of the luer lock cap was measured. Table 2 shows the results. Herein, n is the number of experiments.

**[Table 2]**

| | Present invention | Company A | Company B | Company C |
|---|---|---|---|---|
| Resistance to water pressure (MPa) | 0.200 to 0.250 (n=10) | 0.015 to 0.020 (n=3) *There are products that almost peel off | <0.010 (n=3) *There are products of water leakage | 0.01 (n=3) |

As shown in the table, the resistance to water pressure of the product of the present invention is 0.20 MPa to 0.25 MPa, and it was confirmed that the product of the present invention had a resistance to water pressure higher by one order or more than those of the companies A, B, and C. In the products of the company A, some of the products having insufficient adhesion were found, and films almost peeled off even under a low water pressure. The product of the company B had a low resistance to water pressure, and liquid infiltrated immediately through the filter. The leakage in the products of the companies A, B, and C under this water pressure cannot necessarily be considered to be sufficient from a practical point of view, and it is rather difficult to adopt those products because applications thereof are limited in medial practice. In contrast, the product of the present invention having the resistance to water pressure shown above can be considered to have practically sufficient reliability, and peeling and water leakage are not found. Thus, it is considered that highly excellent results are obtained.

Considering the above-mentioned results of breathability and resistance to water pressure, it can be concluded that only the product of the present invention is a product with high reliability satisfying both the performances.

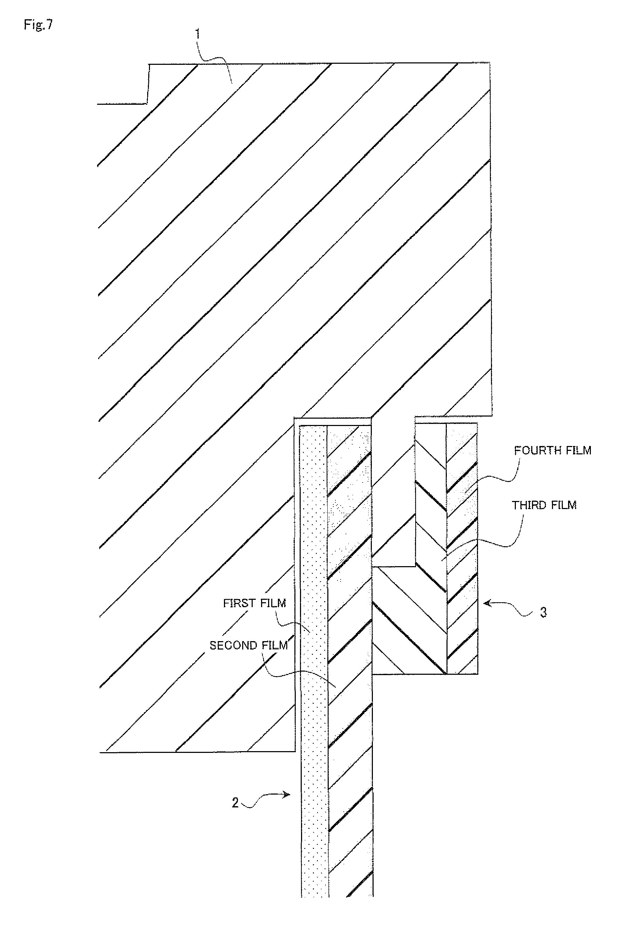

In the above-mentioned example, an embodiment in which the auxiliary film body is set larger than the breathable film to ensure an area where the auxiliary film body is heat-laminated to the luer lock cap is described. However, as long as the auxiliary film body can be heat-laminated to the luer lock cap while being heat-laminated to the breathable film, the present invention is not particularly limited to this embodiment. For example, the breathable structure as illustrated in FIG. 7 may be used.

### INDUSTRIAL APPLICABILITY

The present invention can also be used for the case where the breathable structure is provided in an end surface of a breathable needle to send air to an infusion bag hygienically.

### Brief Description of Drawings

[FIG. 1] FIG. 1 is a cross-sectional view of a luer lock cap according to an embodiment of the present invention.
[FIG. 2] FIG. 2 is an enlarged schematic view of a connection portion.
[FIG. 3] FIG. 3 is a conceptual diagram illustrating a state of actual heat lamination.
[FIG. 4] FIG. 4 is a plan view illustrating a state of lamination of an auxiliary film body of the luer lock cap according to the embodiment.
[FIG. 5] FIG. 5 is an experimental general outline view of an evaluation test of breathability.
[FIG. 6] FIG. 6 is an experimental general outline view of an evaluation test of resistance to water pressure.
[FIG. 7] FIG. 7 is a partially cross-sectional view of a luer cap lock having another breathable structure.

### Reference Signs List

- 1: luer lock cap
- 2: breathable film
- 3: auxiliary film body
- 11: through-hole
- 21: first film
- 22: second film
- 31: third film
- 32: fourth film
- 33: punched hole

## Claims

1. A breathable structure, which is adopted in a luer lock cap to be connected to a path of a medical tube body or in other housings, comprising:
a breathable film;
a base portion that is a housing portion in which the breathable film is provided; and
an auxiliary film body assisting in connection between the breathable film and the base portion, wherein:
the base portion is made of synthetic resin having a low melting point, comprising a through-hole to be covered with the breathable film;
the breathable film comprises a two-layered film made of synthetic resin having breathability, in which a second film having a low melting point adheres to a porous first film having water repellency;
the auxiliary film body comprises a two-layered film made of synthetic resin, in which a fourth film having a melting point higher than a melting point of a third film having a low melting point adheres to the third film;
the breathable film is connected to the auxiliary film body with an inner peripheral portion of the third film being heat-laminated to the second film; and
the auxiliary film body is connected to the base portion with an outer peripheral portion of the third film being heat-laminated to the base portion.

2. A breathable structure, which is adopted in a luer lock cap to be connected to a path of a medical tube body or in other housings, comprising:
a breathable film;
a base portion that is a housing portion in which the breathable film is provided; and
an auxiliary film body assisting in connection between the breathable film and the base portion, wherein:
the base portion is made of synthetic resin comprising a low melting point, comprising a through-hole to be covered with the breathable film;
the breathable film comprises a two-layered film made of synthetic resin having breathability, in which a second film having a low melting point adheres to a porous first film having water repellency;
the auxiliary film body comprises a two-layered film made of synthetic resin with an outer shape larger than an outer shape of the breathable film, in which a fourth film having a melting point higher than a melting point of a third film having a low melting point adheres to the third film;
the breathable film is connected to the auxiliary film body with the second film being heat-laminated to the third film so that the breathable film does not extend beyond the auxiliary film body;
and
the auxiliary film body is connected to the base portion with an outer edge portion of the third film being heat-laminated to the base portion.

3. A breathable structure according to claim 2, wherein the base portion comprises, on a surface thereof:
a first recess having substantially the same shape as the outer shape of the auxiliary film body with the through-hole being a center; and
a second recess having substantially the same shape as the outer shape of the breathable film at a center of the first recess.

4. A breathable structure according to claim 1, 2, or 3, wherein the breathable film is obtained by heat-laminating non-weven fabric of polyolefin to a porous PTFE sheet.

5. A breathable structure according to any one of claims 1 to 4, wherein the second film, the third film, and the base portion are made of polyolefin.

6. A breathable structure according to any one of claims 1 to 5, wherein the auxiliary film body is formed by punching out a hole having a shape smaller than the outer shape of the breathable film from the auxiliary film body at a center thereof.

7. A method of forming a breathable structure, which is adopted in a luer lock cap to be connected to a path of a medical tube body or in other housings, the breathable structure comprising:
a breathable film that is a two-layered film made of synthetic resin having breathability, in which a second film having a low melting point adheres to a porous first film having water repellency;
a base portion made of synthetic resin having a low melting point, which is a housing portion comprising a through-hole to be covered with the breathable film; and
an auxiliary film body that is a two-layered film made of synthetic resin, in which a fourth film having a melting point higher than a melting point of a third film having a low melting point adheres to the third film, and which assists in connection between the breathable film and the base portion and has an outer shape larger than an outer shape of the breathable film,
the method comprising:
placing the breathable film on the through-hole so that the first film side faces the through-hole, and further placing the auxiliary film body on the breathable film so that the fourth film side is directed upward and the breathable film does not extend beyond the auxiliary film body; and
pressing heating means from outside of the housing to heat-laminate the second film to the third film and the third film to the base portion.

8. A method of forming a breathable structure according to claim 7, comprising:
providing a first recess having substantially the same shape as the outer shape of the auxiliary film body on a surface of the base portion with the through-hole being a center, and providing a second recess having substantially the same shape as the outer shape of the breathable film at a center of the first recess;
placing the breathable film on the second recess so that the first film side is directed downward;
placing the auxiliary film body on the first recess so that the fourth film side is directed upward; and
pressing the heating means from the fourth film side to heat-laminate the second film to the third film and the third film to the base portion.

9. A method of forming a breathable structure according to claim 7 or 8, comprising:
firstly heat-laminating the third film to the base portion; and
secondly heat-laminating the second film to the third film.

10. A method of forming a breathable structure, which is adopted in a luer lock cap to be connected to a path of a medical tube body or in other housings, the breathable structure comprising:
a breathable film that is a two-layered film made of synthetic resin having breathability, in which a second film having a low melting point adheres to a porous first film having water repellency;
a base portion made of synthetic resin having a low melting point, which is a housing portion comprising a through-hole to be covered with the breathable film; and
an auxiliary film body that is a two-layered film made of synthetic resin, in which a fourth film having a melting point higher than a melting point of a third film having a low melting point adheres to the third film, and which assists in connection between the breathable film and the base portion and has an outer shape larger than an outer shape of the breathable film,
the method comprising:
placing a laminate, in which the second film of the breathable film adheres to the third film of the auxiliary film body so that the breathable film does not extend beyond the auxiliary film body, on the through-hole so that the first film side faces the through-hole; and
pressing heating means from outside of the housing to heat-laminate the third film to the base portion.

11. A method of forming a breathable structure according to claim 10, comprising:
providing a recess having substantially the same shape as the outer shape of the auxiliary film body on a surface of the base portion with the through-hole being a center;
placing the auxiliary film body on the recess so that the fourth film side is directed upward; and
pressing the heating means from the fourth film side to heat-laminate the third film to the base portion.

12. A method of forming a breathable structure according to claim 11, comprising:
further providing a second recess having substantially the same shape as the outer shape of the breathable film in the recess so that the through-hole is positioned at a center of the second recess;
placing the breathable film on the second recess so that the first film side is directed downward; and
pressing the heating means from the fourth film side to heat-laminate the third film to the base portion.

13. A method of forming a breathable structure according to any one of claims 7 to 12, wherein the breathable film is obtained by heat-laminating non-woven fabric of polyolefin to a porous PTFE sheet.

14. A method of forming a breathable structure according to any one of claims 7 to 13, wherein the second film, the third film, and the base portion are made of polyolefin.
